# EUROPEAN PATENT APPLICATION

(11) **EP 4 509 898 A1**
(43) Date of publication of application: **19.02.2025**
(21) Application number: 23839257.5
(22) Date of filing: 17.04.2023
(51) Int. Cl.: G02B 21/36, H04N 23/60

(54) **CELL OBSERVATION DEVICE AND IMAGING METHOD USED IN CELL OBSERVATION DEVICE**

(30) Priority: 15.07.2022 JP 2022113571
(71) Applicant: Kataoka Corporation, Kyoto-shi, Kyoto 601-8203 (JP)
(72) Inventor: KUSAKA, Ayumi, Kyoto-shi, Kyoto 601-8203 (JP); MORISHITA, Tadao, Kyoto-shi, Kyoto 601-8203 (JP)
(74) Representative: Seemann & Partner Patentanwälte mbB
(86) International application number: PCT/JP2023/015268
(87) International publication number: WO 2024/014079

(57) **Abstract**

A cell observation apparatus capable of acquiring a taken image in which the focus shift is prevented even in a divided region affected by a meniscus is provided.

A cell observation apparatus of the present invention including:
a placement unit on which a cell culture vessel containing a cell, as an object to be observed, is placed;
an imaging optical system that forms an observation image of the object to be observed;
a movement unit that moves at least one of the placement unit and the imaging optical system along an optical axis direction of the imaging optical system from a movement start position to a movement end position;
an imaging unit that takes a plurality of images of the observation image in movement in the optical axis direction using an image sensor;
an evaluation unit 513 that evaluates a distribution degree of luminance values for a plurality of taken images; and
a selection unit 514 that selects a taken image with the highest distribution degree of luminance values evaluated by the evaluation unit 513 as an in-focus image, in which,
the selection unit 514 selects the in-focus image based only on a distribution degree of luminance values not more than a predetermined luminance value.

## Description

### TECHNICAL FIELD

The present invention relates to a cell observation apparatus and an imaging method used in the cell observation apparatus.

### BACKGROUND ART

When taking an image of the whole surface of one cell culture vessel (for example, a culture dish) using an optical cell observation apparatus such as a bright-field microscope or a phase-contrast microscope, the culture vessel is divided into a plurality of fractions (divided regions) and an image of each divided region is taken to acquire a divided image. Then, the acquired images are integrated to create an integrated image that integrates observation images of the whole surface of the culture vessel.

When taking the image for each divided region of the culture vessel, the optical axis is shifted around the sidewall of the culture vessel due to the lens action of the meniscus. Thus, in the divided images taken using the cell observation apparatus, there is an issue that a divided image that is not focused on an object to be observed, that is, an out-of-focus image, is generated (Patent Literature 1).

### CITATION LIST

### PATENT LITERATURE

Patent Literature 1: WO 2016/084551 A1

### SUMMARY OF INVENTION

### TECHNICAL PROBLEM

In order to avoid the meniscus effect, the optical observation apparatus of Patent Literature 1, which includes an adjustment optical system that adjusts the shift of the optical axis, adjusts the shift of the optical axis using the adjustment optical system. However, the optical observation apparatus of Patent Literature 1 requires a new optical system to be added to the optical observation apparatus.

Hence, the present invention is intended to provide a cell observation apparatus capable of acquiring a taken image in which the focus shift is prevented even in a divided region affected by a meniscus.

### SOLUTION TO PROBLEM

**In** order to achieve the above object, the present invention provides a cell observation apparatus (hereinafter also referred to as "observation apparatus"), including:
a placement unit on which a cell culture vessel containing a cell, as an object to be observed, is placed;
an imaging optical system that forms an observation image of the object to be observed;
a movement unit that moves at least one of the placement unit and the imaging optical system along an optical axis direction of the imaging optical system from a movement start position to a movement end position;
an imaging unit that takes a plurality of images of the observation image using an image sensor in movement in the optical axis direction;
an evaluation unit that evaluates a distribution degree of luminance values for a plurality of taken images; and
a selection unit that selects a taken image with the highest distribution degree of luminance values evaluated by the evaluation unit as an in-focus image,
wherein the selection unit selects the in-focus image based only on a distribution degree of luminance values not more than a predetermined luminance value.

An imaging method used in a cell observation apparatus including a placement unit, an imaging optical system, a movement unit, and an imaging unit, the imaging method including:
an image forming step of, for a cell culture vessel containing a cell that is placed on the placement unit as an object to be observed, forming an image of the object to be observed using the imaging optical system that forms an observation image of the object to be observed;
a moving step of moving at least one of the placement unit and the imaging optical system along an optical axis direction of the imaging optical system from a movement start position to a movement end position;
an imaging step of taking a plurality of images of the observation image in movement in the optical axis direction using an image sensor of the imaging unit;
an evaluating step of evaluating a distribution degree of luminance values for a plurality of taken images; and
a selecting step of selecting a taken image with the highest distribution degree of luminance values evaluated by the evaluation unit as an in-focus image, wherein
in the selecting step, the in-focus image is selected based only on a distribution degree of luminance values not more than a predetermined luminance value.

### ADVANTAGEOUS EFFECTS OF INVENTION

According to the present invention, it is capable of acquiring a taken image in which the focus shift is prevented even in a divided region affected by the meniscus.

### BRIEF DESCRIPTION OF DRAWINGS

FIG. 1 is a schematic cross-sectional view illustrating an example of an observation apparatus according to a first embodiment.
FIG. 2 is a block diagram illustrating an example of an arithmetic unit in the observation apparatus according to the first embodiment.
FIG. 3 is a diagram illustrating a relationship among information on phases of a drive signal received by a pulse counter, the count by the pulse counter, an imaging trigger signal, and imaging by a camera, according to the first embodiment.
FIG. 4 is a flowchart illustrating an imaging method according to the first embodiment.
FIG. 5 illustrates an example of histograms of luminance values of taken images taken using the camera, according to the first embodiment.
FIG. 6 is a schematic diagram illustrating an example of histograms of luminance values of a plurality of taken images taken at different positions in a Z-axis direction, according to the first embodiment.
FIG. 7 illustrates photographs showing results of selecting images closer to the focal position based on a variance value of luminance values or a distribution degree of luminance values from the plurality of taken images in the Z-axis direction taken in a region affected by the meniscus, according to the first embodiment.
FIG. 8 illustrates in-focus images taken in a region unaffected by the meniscus and regions where the meniscus effect differs, and histograms of luminance values of the in-focus images, according to the first embodiment.
FIG. 9 illustrates in-focus images taken under conditions with different intensities of illumination light from an illumination, and histograms of luminance values of the in-focus images, according to the first embodiment.
FIG. 10 illustrates in-focus images when there is dirt on a cell culture vessel and when there is no dirt thereon, and histograms of luminance values of the in-focus images, according to the first embodiment.
FIG. 11 is a schematic diagram illustrating an example of histograms of luminance values of a plurality of taken images taken at different positions in the Z axis direction, according to a second embodiment.
FIG. 12 is a block diagram illustrating an example of a configuration of an observation apparatus 300 according to a third embodiment.
FIG. 13 is a flowchart illustrating an imaging method according to the third embodiment.
FIG. 14 illustrates an example of histograms of luminance values of taken images taken using a camera, according to the third embodiment.

### DESCRIPTION OF EMBODIMENTS

The term "optical axis direction" as used herein means the direction of the optical axis (symmetrical axis) in the imaging optical system, also referred to as the "Z-axis direction". The optical axis direction can also refer to, for example, a direction orthogonal (perpendicular) to the surface on which the object to be observed is placed. In addition, in the present invention, the term "X-axis direction" refers to one direction on a plane (XY plane) orthogonal to the optical axis direction, and the term "Y-axis direction" means a direction orthogonal (perpendicular) to the X-axis direction on the XY plane.

The term "observation" as used herein means observation of an object to be observed, which may be, for example, observation with or without imaging.

The term "cell" as used herein means a cell or a composition comprising a cell. The cell may be, for example, a cell, a cell aggregate composed of cells, a tissue, an organ, or the like. The cell may be, for example, a cultured cell or a cell isolated from a living body.

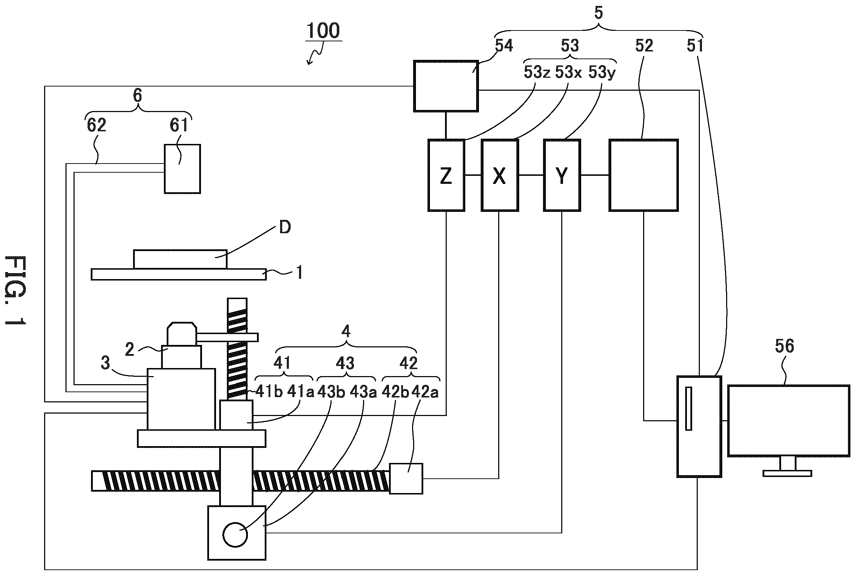

Hereinafter, the observation apparatus according to the present invention will be described in detail with reference to the drawings. The present invention, however, is not limited by the following description. Note that in FIGs. 1 to 7 below, the same parts are denoted by the same reference numerals, and the description thereof may be omitted. Furthermore, in the drawings, the structure of each component may be illustrated in a simplified manner as appropriate for convenience of the description, and the size, the ratio, and the like of each component may be schematically illustrated and different from actual ones. Unless otherwise stated, descriptions regarding the respective embodiments are applicable to each other.

### (First Embodiment)

A first embodiment relates to an observation apparatus and an imaging method of the present invention.

The present embodiment is an example of the observation apparatus. FIG. 1 is a schematic view illustrating an observation apparatus 100 of the first embodiment. As illustrated in FIG. 1, the observation apparatus 100 includes, as its main components, a stage 1 which serves as a placement unit, an objective lens 2 which serves as an imaging optical system, a camera 3 which serves as an imaging unit, a movement unit 4, a control unit 5, and an illumination 6. The movement unit 4 includes a Z-axis direction movement unit 41 which serves as a first movement unit, and an X-axis direction movement unit 42 and a Y-axis direction movement unit 43 which serve as a second movement unit. The Z-axis direction movement unit 41 includes a Z-axis motor 41a and a Z-axis ball screw 41b. The X-axis direction movement unit 42 includes an X-axis motor 42a and an X-axis ball screw 42b. The Y-axis direction movement unit 43 includes a Y-axis motor 43a and a Y-axis ball screw 43b. The control unit 5 includes an arithmetic unit 51, a sequencer (PLC) 52, a motor driver 53, and a pulse counter 54. A configuration of the arithmetic unit 51 will be described below. The motor driver 53 includes an X-axis direction motor driver 53x, a Y-axis direction motor driver 53y, and a Z-axis direction motor driver 53z. The illumination 6 includes a light source 61 and a support member 62 that supports the light source 61.

On the stage 1, a cell culture vessel D containing a cell, which is a configuration outside the observation apparatus 100, is placed. As the stage 1, any configuration on which the object to be observed can be placed may be employed. As a specific example, a configuration of a placement unit in an optical observation apparatus can be used for the placement unit. Examples of the optical observation apparatus include a bright-field microscope, a stereoscopic microscope, a phase-contrast microscope, a differential interference microscope, a polarizing microscope, a fluorescence microscope, a confocal laser microscope, a total internal reflection fluorescence microscope, a Raman microscope, and the like, and preferably, the apparatus is a phase-contrast microscope. In the stage 1, the placement region for the cell culture vessel D is configured so that the cell culture vessel D can be observed through the objective lens 2 arranged below the stage 1. The placement region for the cell culture vessel D may be formed of a translucent material such as glass, quartz, plastic or resin, or a through hole may be formed in a part thereof.

The object to be observed is an object to be observed by the observation apparatus 100. Although in the present embodiment, the object to be observed is the cell culture vessel D containing a cell, the object to be observed can be any sample that can be observed by the optical observation apparatus. Examples of the object to be observed include a cell culture vessel such as a dish, plate, or flask (cell culture flask) containing a cell, or a preparation on which the sample is placed.

The objective lens 2 forms an observation image of the cell culture vessel D which is the object to be observed on the camera 3 which serves as an image sensor. More specifically, the objective lens 2 forms an observation image of a cell in the cell culture vessel D on the camera 3. Thus, the observation apparatus 100 enables observation and imaging of the cell in the cell culture vessel D. Although in the observation apparatus 100, the imaging optical system is included as the objective lens 2, the imaging optical system only needs to be capable of forming the observation image of the object to be observed. For example, a configuration of an imaging optical system in the above-described optical observation apparatus can be employed, for the imaging optical system. In the observation apparatus 100, the number of the objective lenses 2 is one, but may be two or more. In this case, the magnification of each objective lens 2 may be the same or different.

Although in the observation apparatus 100, the objective lens 2 is arranged below the cell culture vessel D, the objective lens 2 may be arranged above the cell culture vessel D. The arrangement location of the objective lens 2 which serves as the imaging optical system can be set as appropriate according to, for example, the type of the above-described optical observation apparatus.

The camera 3 is capable of taking an observation image of the cell culture vessel D which is the object to be observed, and more specifically, the camera 3 is configured to be capable of taking an observation image of a cell in the cell culture vessel D. Although in the observation apparatus 100, the camera 3 including the image sensor is used as the imaging unit, any configuration capable of taking the observation image of the object to be observed can be employed. For example, a known image sensor can be used for the image sensor, and specific examples thereof include devices such as a charge-coupled device (CCD), a complementary metal oxide semiconductor (CMOS). Thus, for the imaging unit, a camera or the like including any of these image sensors can be employed, for example.

The camera 3 is configured to take an observation image of the cell culture vessel D which is the object to be observed upon receiving an imaging trigger signal transmitted from the pulse counter 54 to be described below. A period of one imaging time (exposure time) of the camera 3 can be set as appropriate according to, for example, the brightness of the object to be observed.

In the observation apparatus 100, after the observation image is formed on the camera 3, the observation image is taken by the camera 3 and the acquired image is displayed on a display device 56 outside the apparatus. In addition to displaying the image on the display device 56, the observation apparatus 100 may relay the image acquired by the objective lens 2 (primary image) onto an eyepiece, through which a user of the observation apparatus 100 observes. In this case, the observation apparatus 100 includes, for example, an eyepiece and a relay optical system that relays the primary image onto the eyepiece. For the relay optical system and the eyepiece, a configuration of a relay optical system and an eyepiece in the above-described optical observation apparatus can be used, for example. Specific examples of the display device 56 will be described below.

The camera 3 which serves as the imaging unit transmits the taken image to the arithmetic unit 51. In this case, it is preferable that the camera 3 associate, with the taken image, an imaging position of the image (for example, coordinates such as XYZ coordinates) and transmit it to the arithmetic unit 51.

The movement unit 4 is capable of moving the objective lens 2 which serves as the imaging optical system, and the camera 3 which serves as the image sensor. Although in the observation apparatus 100, the movement unit 4 is capable of moving the objective lens 2 and the camera 3, the movement unit 4 may be configured to be capable of moving only the objective lens 2. In addition to, or instead of the objective lens 2, the movement unit 4 may be configured to be capable of moving the stage 1 which serves as the placement unit. In this case, the stage 1 may have a configuration in which the stage 1 and the movement unit 4 are integrated, such as a motorized stage, or a mechanical stage. The configuration of the movement unit 4 capable of moving the objective lens 2 which serves as the imaging optical system can reduce fluctuations in the liquid surface of the culture solution in the cell culture vessel D which is the object to be observed, and the like, as compared with a case of configuration capable of moving the stage 1. Thus, the observation apparatus 100 can prevent the occurrence of fluctuations in the intensity of the illumination light, variations of the focal position, and the like caused by the fluctuations in the liquid surface. This enables the camera 3 to acquire a plurality of taken images including an image that is in focus (in-focus image) with higher accuracy.

The movement unit 4 includes the first movement unit that is movable in the Z-axis direction, that is, the optical axis direction, and the second movement unit that is movable on the XY plane direction. In the movement unit 4, the first movement unit includes the Z-axis motor 41a and the Z-axis ball screw 41b. In addition, the second movement unit includes the X-axis motor 42a and the X-axis ball screw 42b, and the Y-axis motor 43a and the Y-axis ball screw 43b. As illustrated in FIG. 1, the Z-axis ball screw 41b, the X-axis ball screw 42b, and the Y-axis ball screw 43b are mounted so as to align with the Z-axis direction, the X-axis direction, and the Y-axis direction, respectively. Furthermore, the Z-axis ball screw 41b is connected to a nut on the X-axis ball screw 42b to be movable in the X-axis direction along the X-axis ball screw 42b. The X-axis ball screw 42b is connected to a nut on the Y-axis ball screw 43b to be movable in the Y-axis direction along the Y-axis ball screw 43b. The objective lens 2 and the camera 3 are connected to a nut on the Z-axis ball screw 41b to be movable in the Z-axis (optical axis) direction along the Z-axis ball screw 41b. Then, the Z-axis motor 41a, the X-axis motor 42a, and the Y-axis motor 43a are connected to the Z-axis direction motor driver 53z, the X-axis direction motor driver 53x, and the Y-axis direction motor driver 53y, respectively. In the observation apparatus 100, a drive signal to be described below is transmitted by the motor driver 53. Then, the Z-axis motor 41a, the X-axis motor 42a, and the Y-axis motor 43a are driven based on the drive signal, and thus the objective lens 2 and the camera 3 move to the XYZ coordinates specified by the drive signal. Examples of the Z-axis motor 41a, the X-axis motor 42a, and the Y-axis motor 43a include a motor capable of position control, and as a specific example, a stepping motor can be used therefor.

In the observation apparatus 100, the movement unit 4 only needs to be capable of moving at least one of the placement unit and the imaging optical system, and the corresponding configuration in the above-described optical observation apparatus can be used therefor. Although the movement unit 4 includes the ball screws and the motors, the movement unit 4 may include a linear motor, and may be further combined with a carriage. In a case where the objective lens 2 includes a focus adjustment mechanism such as a lens extension mechanism, the focus adjustment mechanism may be used as the first movement unit in the movement unit 4.

The movement unit 4 only needs to be configured to be capable of moving, in the optical axis (Z-axis) direction, at least one of the placement unit and the imaging optical system, and may be further configured to be movable in at least one of the X-axis direction and the Y-axis direction. The movement in the X-axis direction and the Y-axis direction may be referred to as the movement on a plane (XY plane) in the direction perpendicular (orthogonal) to the optical axis direction, for example.

The control unit 5 includes, as described above, the arithmetic unit 51, the PLC 52, the motor driver 53, and the pulse counter 54. In the observation apparatus 100, the arithmetic unit 51 in the control unit 5 cooperates with the PLC 52, the motor driver 53, and the pulse counter 54 to function as respective units such as a movement control unit including a movement instruction unit 511, an imaging control unit including an imaging instruction unit 512, an evaluation unit 513, and a selection unit 514. The present invention is not limited thereto, and the arithmetic unit 51 may independently function as the movement control unit, the imaging control unit, the evaluation unit 513, and the selection unit 514, or may cooperate with the motor driver 53 and the pulse counter 54 to function as the movement control unit, the imaging control unit, the evaluation unit 513, and the selection unit 514.

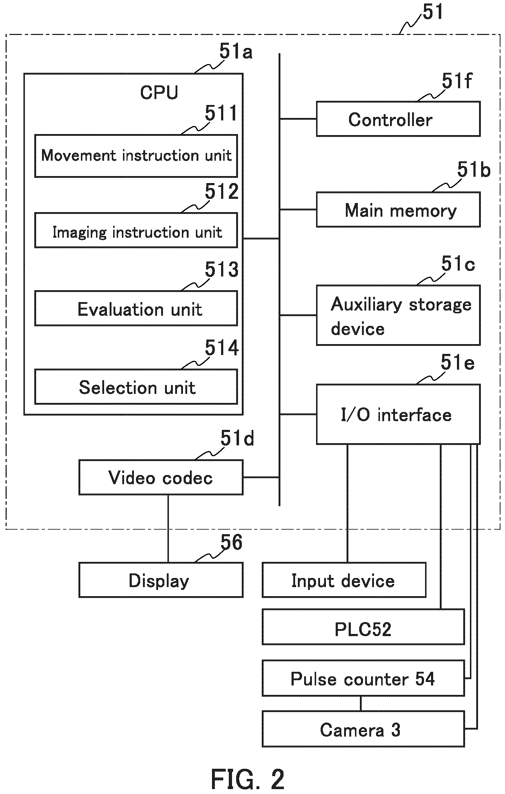

The arithmetic unit 51 has a configuration similar to a personal computer, a server computer, a workstation, or the like. FIG. 2 is a block diagram illustrating an example of the arithmetic unit 51 in the observation apparatus 100. As illustrated in FIG. 2, the arithmetic unit 51 includes a central processing unit (CPU) 51a, a main memory (main storage device) 51b, an auxiliary storage device 51c, a video codec 51d, an input-output (I/O) interface 51e, and the like, which are controlled by a controller (a system controller, an I/O controller, or the like) 51f and operate in cooperation with each other. Examples of the auxiliary storage device 51c include a storage means such as a flash memory, a hard disk drive, or the like. Although the arithmetic unit 51 includes the CPU 51a as a computational means, the arithmetic unit 51 may include a GPU to be described below. The video codec 51d includes a graphics processing unit (GPU) that generates a screen to be displayed based on a drawing instruction received from the CPU 51a and transmits the screen signal to, for example, the display device 56 outside the observation apparatus 100 and the like, a video memory that temporarily stores the screen and image data, and the like. The I/O interface 51e is a device that is communicably connected to the PLC 52, the pulse counter 54, and the camera 3 to control them or acquire information on images and the like. The I/O interface 51e may include a servo driver (servo controller). In addition, the I/O interface 51e may be connected to an input means (input device) outside the observation apparatus 100, for example. Examples of the display device 56 include a monitor that outputs images (for example, various image display devices such as a liquid crystal display (LCD) and a cathode ray tube (CRT) display). Examples of the input device include a touch panel, a track pad, a pointing device such as a mouse, a keyboard, and a push button that can be operated by a finger of the user.

The programs executed by the arithmetic unit 51 and the respective pieces of information are stored in the auxiliary storage device 51c. The programs are read into the main memory 51b at the time of executing the programs and are decoded by the CPU 51a. The arithmetic unit 51 functions as respective units, such as the movement instruction unit 511, the imaging instruction unit 512, the evaluation unit 513, and the selection unit 514 according to the programs. Thus, in the observation apparatus 100, the CPU 51a functions as the movement instruction unit 511, the imaging instruction unit 512, the evaluation unit 513, and the selection unit 514. Functions of the respective units will be described below.

As illustrated in FIG. 1, the PLC 52 is connected to the motor driver 53, and more specifically, is connected to the Z-axis direction motor driver 53z, the X-axis direction motor driver 53x, and the Y-axis direction motor driver 53y. For the PLC 52, a programmable logic controller (PLC) or a sequencer can be used, for example. The PLC52 converts information specifying a movement position of the objective lens 2 transmitted by the arithmetic unit 51 to a motor command for the motor driver 53 that controls the Z-axis motor 41a, the X-axis motor 42a, and Y-axis motor 43a. The PLC 52 then drives the Z-axis motor 41a, the X-axis motor 42a, and the Y-axis motor 43a via the motor driver 53, and when the objective lens 2 reaches the specified movement position, the PLC 52 transmits, to the arithmetic unit 51, information that the movement of the objective lens 2 is completed.

In the observation apparatus 100, the arithmetic unit 51 controls the motor driver 53 via the PLC 52. That is, the movement instruction unit 511 of the arithmetic unit 51, the PLC 52, and the motor driver 53 cooperate with each other to function as the movement control unit that controls the movement by the movement unit 4. The present invention, however, is not limited thereto, and the arithmetic unit 51 may directly control the motor driver 53. In this case, the arithmetic unit 51 includes, for example, a motor controller; a microcontroller with a motor control function, a field-programmable gate array (FPGA);or the like.

As illustrated in FIG. 1, the motor driver 53 includes the Z-axis direction motor driver 53z, the X-axis direction motor driver 53x, and the Y-axis direction motor driver 53y. The Z-axis direction motor driver 53z, the X-axis direction motor driver 53x, and the Y-axis direction motor driver 53y are connected to the Z-axis motor 41a, the X-axis motor 42a, and the Y-axis motor 43a, respectively. Then, the Z-axis direction motor driver 53z, the X-axis direction motor driver 53x, and the Y-axis direction motor driver 53y each transmit the drive signal based on the motor command transmitted from the PLC 52 to drive the Z-axis motor 41a, the X-axis motor 42a, and the Y-axis motor 43a, respectively. The drive signal is, for example, a bi-phasic pulse signal. In addition, the Z-axis direction motor driver 53z is connected to the pulse counter 54. Thus, in addition to the transmission of the drive signal to the Z-axis motor 43a, the Z-axis direction motor driver 53z also transmits the drive signal to the pulse counter 54.

As illustrated in FIG. 1, the pulse counter 54 is connected to the camera 3. For the pulse counter 54, a microcontroller or the like can be used. Upon receiving an ON signal from the imaging instruction unit 512 of the arithmetic unit 51, the pulse counter 54 starts the count of the number of drive signal transmissions from the Z-axis direction motor driver 53z. Then, when the count reaches a predetermined number of times, the pulse counter 54 transmits the imaging trigger signal to command the camera 3 to take an image. The predetermined number of times can be set as appropriate based on, for example, the movement distance in the optical axis direction of the camera 3 when the Z-axis motor 41a receives the drive signals the predetermined number of times (the predetermined distance). The movement distance in the optical axis direction is, for example, 1 to 50 µm. By shortening the movement distance in the optical axis direction, the observation apparatus 100 can acquire the plurality of taken images including the image that is in focus on the object to be observed, with higher accuracy. Alternatively, by lengthening the movement distance in the optical axis direction, the observation apparatus 100 can acquire the plurality of taken images including the image that is in focus on the object to be observed, with a shorter time. Upon receiving an OFF signal from the imaging instruction unit 512 of the arithmetic unit 51, the pulse counter 54 stops the count of the number of drive signal transmissions. In addition, the pulse counter 54 may also be configured to reset the count (for example, to 0) upon receiving, from the arithmetic unit 51, a signal to reset the count of the number of drive signal transmissions, for example.

In the observation apparatus 100, the arithmetic unit 51 controls the pulse counter 54 via the PLC 52 and the Z-axis direction motor driver 53z. That is, the imaging instruction unit 512 of the arithmetic unit 51, the PLC 52, the Z-axis direction motor driver 53z, and the pulse counter 54 cooperate with each other to function as the imaging control unit that controls imaging by the camera 3. The present invention, however, is not limited thereto, and the arithmetic unit 51 may directly control the imaging by the camera 3. In this case, the arithmetic unit 51 includes, for example, a microcontroller, a field-programmable gate array (FPGA); or the like, and counts the number of drive signal transmissions from the Z-axis direction motor driver 53z, and transmits the imaging trigger signal to command the camera 3 to take an image when the count reaches the predetermined number of times.

Although the pulse counter 54 is connected to the Z-axis direction motor driver 53z, the pulse counter 54 may be connected to the Z-axis motor 41a. In this case, the Z-axis motor 41a includes an encoder such as a linear encoder (linear scale). Then, the pulse counter 54 may receive, as the drive signal, a signal such as a bi-phasic pulse output from the Z-axis motor 41a to count the number of times thereof.

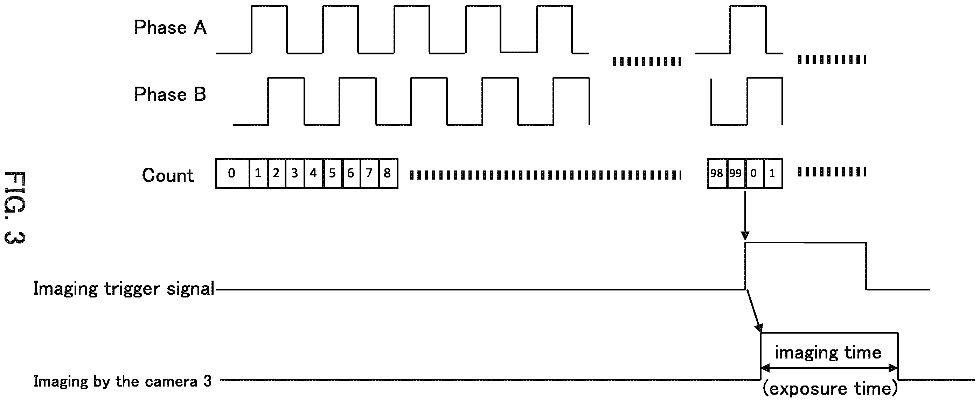

FIG. 3 is used to illustrate how to drive the pulse counter 54 more specifically. FIG. 3 is a diagram illustrating a relationship among information on phases of the drive signal received by the pulse counter 54, the count by the pulse counter 54, the imaging trigger signal, and imaging by the camera 3. An example in the case where the drive signal is a bi-phasic pulse signal including a phase A and a phase B, and the predetermined number of times is 100 times will be described, however, the type of the drive signal and the predetermined number of times are not limited to the example. In addition, although in the following description, an example in which the in-focus image is selected using the plurality of images taken by the camera 3 with a negative contrast optical system will be given, the present invention is not limited to the example, and the in-focus image may be selected using the plurality of images taken by the camera 3 with a positive contrast optical system.

As illustrated in FIG. 3, the drive signal includes the phase A and the phase B with a phase difference. The pulse counter 54 counts using a combination of a rising edge and a falling edge of these phases. Specifically, in FIG. 3, the pulse counter 54 counts once when the phase A rises while the phase B is in the low pulse (L pulse) state, and counts once when the phase B rises while the phase A is in the high pulse (H pulse) state. Furthermore, the pulse counter 54 counts once when the phase A falls while the phase B is in the H pulse state, and counts once when the phase B falls while the phase A is in the L pulse state. Then, when the pulse counter 54 counts one more time in a state where the accumulated count is 99 times, the count reaches the predetermined number of times, so that the pulse counter 54 transmits the imaging trigger signal to the camera 3. In addition, the pulse counter 54 resets the count to 0. Although the example in the case of using the phase A and the phase B to accumulate the counts has been described in FIG. 3, in a case where the drive signal is in opposite phases to the phases A and B in FIG. 3, the pulse counter 54 counts as follows. The pulse counter 54 counts once when the phase A rises while the phase B is in the H pulse state, and counts once when the phase B falls while the phase A is in the H pulse state. Furthermore, the pulse counter 54 counts once when the phase A falls while the phase B is in the L pulse state, and counts once when the phase B rises while the phase A is in the L pulse state. Then, the pulse counter 54 decreases the count from 0, to 99, to 98, and so on, and when the count reaches 0, the pulse counter 54 transmits the imaging trigger signal to the camera 3. Accordingly, the pulse counter 54 detects the movement in both directions in the optical axis direction, which enables imaging by the camera 3.

Next, the illumination 6 illuminates the cell culture vessel D which is the object to be observed, placed on the stage 1. The illumination 6 is an optional configuration and may be present or absent. The illumination 6 includes a light source capable of illuminating the object to be observed. In addition to the light source 61, the illumination 6 may include an illumination optical system that guides the illumination light from the light source to the object to be observed. The light source 61 may be, for example, a halogen lamp, a tungsten lamp, a light emitting diode (LED). For the illumination optical system, a configuration of an illumination optical system in the above-described optical observation apparatus can be employed, for example.

In the observation apparatus 100, the illumination 6 is arranged to face to the objective lens 2 and camera 3, with the stage 1 in between. That is, the illumination 6 is arranged above the stage 1. However, the arrangement location of the illumination 6 is not limited thereto and can be set as appropriate according to the type of the optical observation apparatus, and the illumination 6 may be arranged below the stage 1, for example. The illumination 6 is connected to the camera 3 via the support member 62, and moves in conjunction with the movement of the camera 3, accordingly. However, the configuration of the illumination 6 is not limited thereto. The illumination 6 may be configured to be movable independently of the camera 3, or may not be movable. In a case where the illumination 6 is configured to be movable independently of the camera 3, it is preferable that the illumination 6 move to align coaxially with the camera 3 along the Z-axis. In the observation apparatus 100, by moving in conjunction with the camera 3, the illumination 6 can suitably illuminate an imaging target region of the cell culture vessel D to be observed and imaged by the objective lens 2 and the camera 3. The imaging target region refers to the region to be imaged by the observation apparatus 100.

Next, an imaging method of the first embodiment using the observation apparatus 100 of the first embodiment will be described.

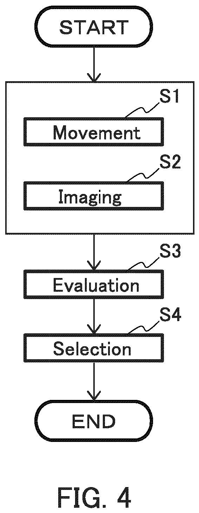

FIG. 4 is a flowchart illustrating the imaging method of the first embodiment. As illustrated in FIG. 4, the imaging method of the first embodiment includes step S1 (movement), step S2 (imaging), step S3 (evaluation), and step S4 (selection). In the imaging method of the first embodiment, step S1 and step S2 are performed in parallel.

In step S1, the objective lens 2 that forms the observation image of the cell culture vessel D which is the object to be observed is moved along the optical axis direction of the objective lens 2 from the movement start position to the movement end position. Specifically, in step S1, the movement instruction unit 511 of the arithmetic unit 51 transmits, to the PLC 52, information (for example, XYZ coordinates) specifying the movement position of the objective lens 2. Then, the PLC 52 and the motor driver 53 cooperate with each other to control the movement by the movement unit 4 based on the information specifying the movement position of the objective lens 2. In step S1, the objective lens 2 is moved from the movement start position to the movement end position in the Z-axis direction by the movement unit 4.

The movement start position refers to the position at which the movement in the optical axis direction starts, and specifically, the position at which the movement in the optical axis direction starts and control to start taking the observation image is executed by the imaging instruction unit 512 to be described below. The movement end position refers to the position at which the movement in the optical axis direction ends, and specifically, the position at which the movement in the optical axis direction ends and control to end taking the observation image is executed by the imaging instruction unit 512 to be described below. The movement start position and the movement end position may be set in advance by the user of the observation apparatus 100, or may be set based on the coordinates of the bottom surface in the container of the cell culture vessel D or the coordinates of the stage 1, for example. The movement start position and the movement end position are expressed, for example, as XYZ coordinates. The distance between the movement start position and the movement end position in the optical axis (Z-axis) direction can be set based on, for example, the thickness of the object to be observed, that is, the length in the optical axis direction thereof. When the object to be observed is a cell, the distance between the movement start position and the movement end position in the optical axis direction is, for example, 0.2 to 0.5 mm. The movement start position and the movement end position can be set, for example, to include a position in the Z-axis direction at which the in-focus image can be acquired in the region unaffected by the meniscus.

Although in the imaging method of the first embodiment, the objective lens 2 which serves as the imaging optical system and the camera 3 are moved in step S1, the present invention is not limited thereto. In step S1, the stage 1 which serves as the placement unit may be moved, or both the stage 1 and the objective lens 2 may be moved.

Next, in step S2, a plurality of images of the observation image is taken by the camera 3 in the movement in the optical axis direction. Specifically, in step S2, the imaging instruction unit 512 of the arithmetic unit 51 transmits the ON signal to the PLC 52, the Z-axis direction motor driver 53z, and the pulse counter 54. Then, based on the ON signal, the PLC 52, the Z-axis direction motor driver 53z, and the pulse counter 54 cooperate with each other to cause the camera 3 to take the plurality of images of the observation image of the cell in the cell culture vessel D in the movement of the objective lens 2 in the optical axis direction in step S1. In step S2, after associating the XYZ coordinates of the objective lens 2 at the time of imaging with each taken image, the camera 3 transmits the plurality of taken images to the arithmetic unit 51, and the plurality of taken images are stored in the auxiliary memory device 51c of the arithmetic unit 51. Although in step S2, the plurality of taken images are stored in the auxiliary storage device 51c of the arithmetic unit 51, the plurality of taken images may be stored in an external storage device outside the observation apparatus 100.

The imaging control unit controls the imaging by the camera 3 so that the plurality of the observation images is taken by the camera 3 in the movement in the optical axis direction. The number of observation images to be taken can be determined based on the movement distance in the optical axis direction and the distance between the movement start position and the movement end position. The number of the observation images to be taken needs to be two or more, and there is no limit on its maximum number. It is preferable that the imaging instruction unit 512 control the camera 3 to take the plurality of observation images by taking the observation image using the image sensor at each movement of the predetermined distance in the movement in the optical axis direction. As a result, the observation apparatus 100 can select the in-focus image with more focus on the object to be observed in the selection by the selection unit 514 to be described below.

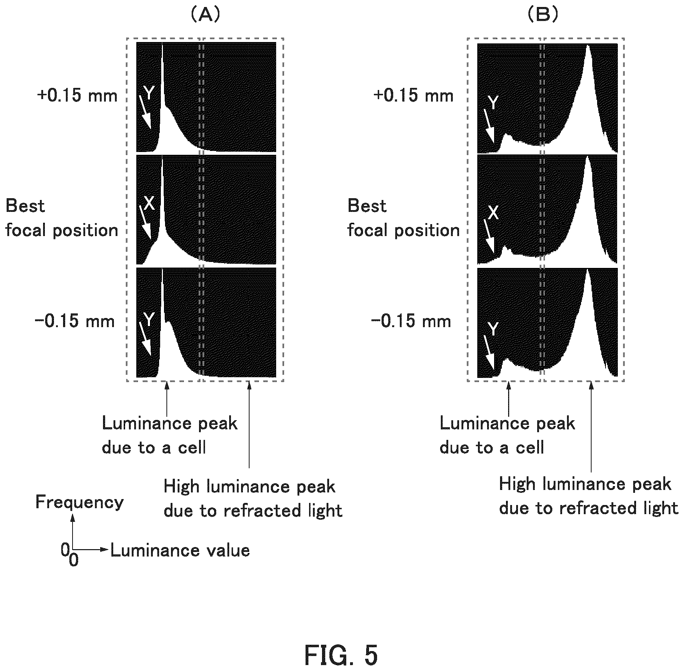

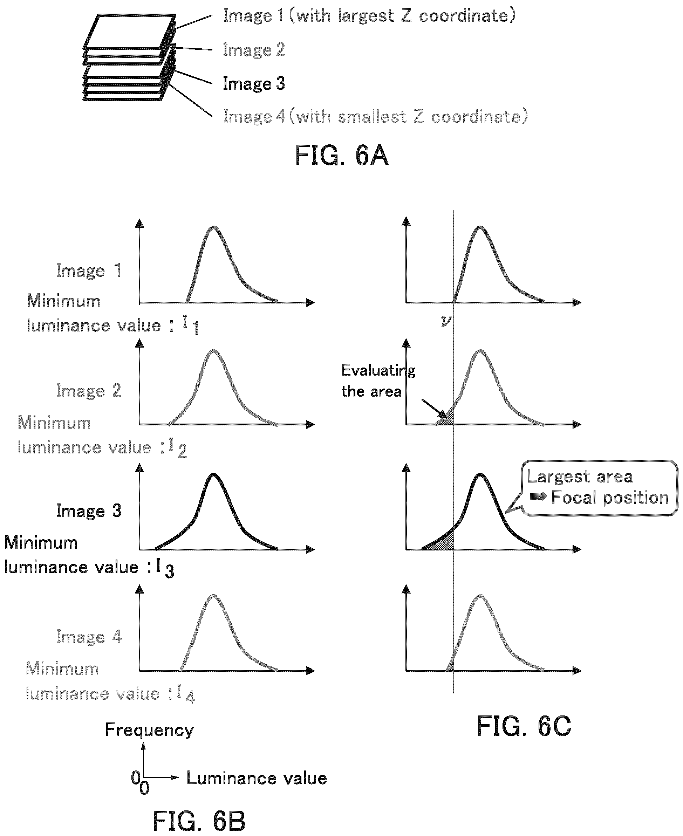

Next, in step S3 and step S4, the in-focus image is selected from the plurality of taken images that is stored in the auxiliary memory device 51c. Step S3 and step S4, which are characteristic processes of the first embodiment, will be described using FIG. 5A to FIG. 5C and FIG. 6A to FIG. 6C. FIG. 5A to FIG. 5C illustrates an example of histograms of luminance values of taken images taken using the camera 3. In FIG. 5A illustrates histograms of luminance values of taken images in a region unaffected by the meniscus, and FIG. 5B illustrates histograms of luminance values of taken images in a region affected by the meniscus. In FIG. 5A and FIG. 5B, respective histograms are, in order from the top, a histogram of luminance values of the taken image taken at a position of a coordinate displaced to the plus side along the Z-axis direction (optical axis direction) from the focal position, a histogram of luminance values of the taken image taken at the focal position, and a histogram of luminance values of the taken image taken at a position of a coordinate displaced to the minus side along the Z-axis direction (optical axis direction) from the focal position. In FIG. 5A to FIG. 5C, the horizontal axis refers to the luminance value and the vertical axis refers to the frequency. Hereinafter, in the other histograms, the parameters of the horizontal axis and the vertical axis are the same thereto.

In the region unaffected by the meniscus, while the luminance peak due to a cell is observed, the high luminance peak due to refracted light caused by the meniscus does not occur, as illustrated in FIG. 5A. On the other hand, in the region affected by the meniscus, in addition to the luminance peak due to the cell, the luminance peak due to the refracted light occurs as illustrated in FIG. 5B. Typically, when selecting the in-focus image from the plurality of taken images acquired at different coordinates in the Z-axis direction, the in-focus image is selected by evaluating the variance value of luminance values. Hence, the in-focus image can be selected in the region unaffected by the meniscus. On the other hand, in the region affected by the meniscus, the in-focus image may not be selected with evaluation using the variance value. This is because in the region affected by the meniscus, there is an effect by the high luminance peak due to the refracted light, and thus, the variance value includes luminance values due to the refracted light. Then, in the region affected by the meniscus, an image that is in-focus is selected among the plurality of taken images based on the variance value including the luminance values due to the refracted light, which may result in inaccurate evaluation.

The present inventors have found that, as indicated by the arrow X in FIG. 5A and FIG. 5B, in the in-focus image, the tail is spread out on the low-luminance side of the luminance peak due to a cell, irrespective of the meniscus effect. On the other hand, the present inventors have found that, as indicated by the arrow Y in FIG. 5A and FIG. 5B, in the taken image acquired at a distance from the focal position, the tail on the low-luminance side becomes relatively narrow compared with that in the in-focus image and little or no spread of the tail is observed when further away from the focal position. It is presumed that in the in-focus image, the resolution of the image is higher and the contrast of luminance values is higher, allowing the regions with low luminance values to be clearly extracted into the taken image, while in the taken image at a distance from the focal position, the resolution of the image decreases and the contrast of luminance values is lower, making it difficult to extract the regions with low luminance values into the taken image. Hence, in the observation apparatus 100 of the first embodiment, the evaluation unit 513 evaluates, for the plurality of taken images, the spread of the tail on the low-luminance side of the luminance peak due to a cell as the distribution degree of luminance values. Then, in the observation apparatus 100 of the first embodiment, an approach is adopted in which the selection unit 514 uses the distribution degree of luminance values acquired by the evaluation unit 513 to select the image with the highest distribution degree of luminance values as the in-focus image. Accordingly, in the observation apparatus 100 of the first embodiment, a taken image closer to the focal position can be accurately selected, irrespective of the meniscus effect.

Next, step S3 will be described in detail using FIG. 6A to FIG. 6C. FIG. 6A to FIG. 6C are a schematic diagram illustrating an example of histograms of luminance values of a plurality of taken images taken at different positions in the Z-axis direction. In FIG. 6A illustrates the positional relationship in the Z-axis direction of the respective taken images, FIG. 6B illustrates the histograms of the respective taken images, and FIG. 6C is a schematic diagram illustrating the number of luminance values not more than a threshold value v. As illustrated in FIG. 6A, the images 1 to 4 are a part of the plurality of taken images, where the image 1 refers to the taken image with the largest Z coordinate, the image 2 refers to the taken image between the image 1 and the image 3, the image 3 refers to the in-focus image, and the image 4 refers to the taken image with the smallest Z coordinate. As described above, in the taken image closer to the focal position, the tail on the low-luminance side of the luminance peak due to a cell is relatively larger, and thus the minimum luminance value I is lower. Then, in step S3, the distribution degree of luminance values is evaluated by, as illustrated in FIG. 6C, setting a threshold value v in luminance values, and counting the area of luminance values not more than or less than the threshold value v in the taken image, that is, the number of pixels with luminance values not more than or less than the threshold value v in the taken image. Although in the present embodiment, the lower limit of the range in which the number of pixels is counted is not determined and the range is, in effect, not less than 0 to not more than (or less than) v, another threshold value µ may be optionally set for the range to be not less than (or more than) µ to not more than (or less than) v.

Specifically, in step S3, the evaluation unit 513 generates, for the taken images (image 1 and image 4 in FIG. 6B) located at both ends in the Z-axis direction, a histogram indicating the distribution of luminance values for all pixels included in the taken image, and extracts the minimum luminance values I₁ and I₄ from the histograms. Next, in step S3, the evaluation unit 513 compares the two minimum luminance values I₁ and I₄ to set the minimum luminance value I₁ with a higher luminance value as the threshold value v. Then, in step S3, the evaluation unit 513 counts, for the plurality of taken images, the number of pixels with luminance values not more than the threshold value v in each taken image. In the calculation of the minimum luminance values I₁ and I₄, it is preferable to remove noise not more than a noise threshold value in advance or to target the region more than the noise threshold value. The noise threshold is, for example, the frequency of a luminance value, which may be set by the user. Although in the present embodiment, the minimum luminance value I from the two taken images located at both ends in the Z-axis direction is set as the threshold value v, the present invention is not limited thereto. The minimum luminance value I of any one of the plurality of taken images may be set as the threshold value v, the minimum luminance values I of any two or more taken images among the plurality of taken images may be compared to set the minimum luminance value I with the highest luminance value as the threshold value v, or the minimum luminance values I of all the plurality of taken images may be compared to set the minimum luminance value I with the highest luminance value as the threshold value v. In addition, although in step S3, the evaluation unit 513 removes the noise, the noise may not be removed. In this case, in step S3, the evaluation unit 513 extracts, for the plurality of taken images, the minimum luminance value I from luminance values for all pixels included in each taken image. Then, in step S3, the minimum luminance values I of the respective taken images are compared to set the minimum luminance value I with the highest luminance value as the threshold value v.

Next, in step S4, the selection unit 514 selects the taken image closer to the focal position (in-focus image) using the numbers of pixels with luminance values not more than the threshold value v of the respective taken images counted in step S3. Specifically, as illustrated in FIG. 6C, in the taken image closer to the focal position, the tail on the low-luminance side of the luminance peak due to a cell is relatively larger, and thus the number of pixels with luminance values not more than the threshold value v is relatively larger. Thus, in step S4, the taken image with the largest number of pixels with luminance values not more than the threshold value v is selected as the taken image with the highest distribution degree of luminance values. In a case where the object to be observed is taken for each of the divided regions, in step S4, the selection unit 514 selects, for the respective divided regions, the taken image with the largest number of pixels with luminance values not more than the threshold value v as the taken image with the highest distribution degree of luminance values.

Next, in a case where the object to be observed is divided into a plurality of regions (divided regions) that can be imaged in a single field of view of the camera 3 and the image of the object to be observed is taken for each of the divided regions, in step S1, the movement instruction unit 511 of the arithmetic unit 51 transmits, to the PLC 52, the information (for example, XYZ coordinates) specifying the movement position of the objective lens 2 for taking an image of the next divided region. The PLC 52 and the motor driver 53 then cooperate with each other to control the movement by the movement unit 4 based on the information specifying the movement position of the objective lens 2, and the objective lens 2 moves to the new divided region. The movement to the new divided region is preferably the movement on the XY plane, and more preferably the movement on the XY plane to an adjacent new divided region. The observation apparatus 100 of the first embodiment performs steps S1 to S4 in the same manner after the movement to the new divided region. Then, the observation apparatus 100 of the first embodiment repeats the movement to a new divided region and the steps S1 to S4 in the new divided region in the same manner until there are no more divided regions that have not been imaged.

After imaging, evaluation and selection of all the divided regions, the observation apparatus 100 of the first embodiment may generate an in-focus image of the entire cell culture vessel D by integrating the in-focus images selected from the plurality of taken images of the respective divided regions. The integration can be performed by arranging the respective in-focus images based on the XYZ coordinates associated with the in-focus image of each divided region. Accordingly, the observation apparatus 100 of the first embodiment can acquire the in-focus image of the entire cell culture vessel D.

Although an example in which the observation apparatus 100 of the first embodiment uses the plurality of taken images taken by the negative contrast optical system, the observation apparatus 100 of the first embodiment may use the plurality of taken images taken by the positive contrast optical system to select the in-focus image. In this case, the observation apparatus 100 of the first embodiment can select the in-focus image by performing the processing in the same manner with replacing, in the descriptions of step S3 and step S4, "not more than" to "not less than", "highest minimum luminance value" to "lowest maximum luminance value", "minimum luminance value" to "maximum luminance value", "with a higher luminance value" to "with a lower luminance value", "more than" to "less than", and "low-luminance side" to "high-luminance side". Note that the same manner can also apply to the other embodiments.

### (Effect of First Embodiment)

In the observation apparatus 100 of the first embodiment, the evaluation unit 513 evaluates, for the plurality of taken images, the spread of the tail on the low-luminance side of the luminance peak due to a cell, which is unaffected by the meniscus and correlates with the focal position, as the number of pixels with luminance values not more than the threshold value v. Then, in the observation apparatus 100 of the first embodiment, the selection unit 514 selects the taken image closer to the focal position (in-focus image) using the number of pixels with luminance values not more than the threshold value v. Thus, the observation apparatus 100 of the first embodiment, which is unaffected by the meniscus, can select the taken image closer to the focal position in both of the region affected by the meniscus and the region unaffected by the meniscus. Since the taken image closer to the focal position can be selected according to the observation apparatus 100 of the first embodiment, the observation apparatus 100 of the first embodiment can be suitably used in combination with an apparatus that uses the selected taken image to perform determination of a cell type, identification of a target for processing, cell treatment or the like. As an example, by combining the observation apparatus 100 of the first embodiment with a laser processing device (laser processing machine), a cell to be removed by laser can be identified with more accuracy, so that the yield reduction caused by laser treatment on a cell not to be removed can be prevented.

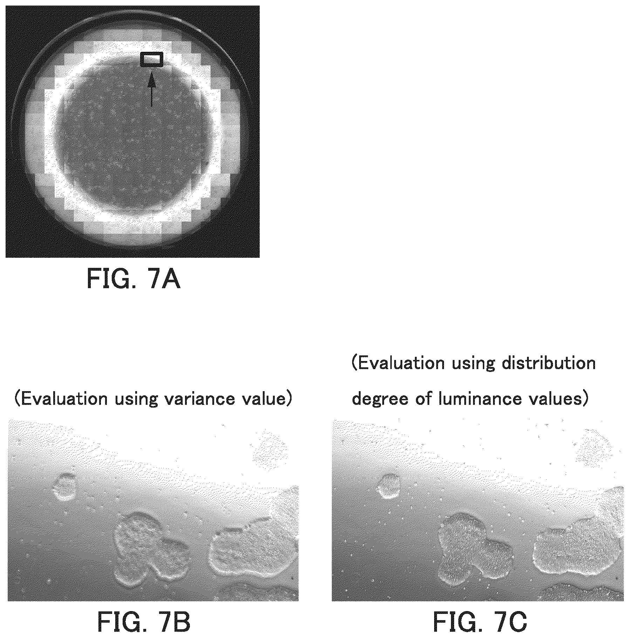

FIG. 7A to FIG. 7C illustrate photographs showing results of selecting images closer to the focal position based on the variance value of luminance values and the distribution degree of luminance values from the plurality of taken images in the Z-axis direction taken in the region affected by the meniscus. **In** FIG. 7A shows the imaging position in the cell culture vessel **D,** FIG. 7B shows the result of selection using the variance value, and FIG. 7C shows the result of selection based on the distribution degree of luminance values. As illustrated in **FIG** 7A, while the center of the cell culture vessel D is unaffected by the meniscus, the periphery part of the cell culture vessel D is affected by the meniscus. As illustrated in FIG. 7B, in a case where the selection is based on the variance value, the taken image that is out-of-focus is selected. On the other hand, as illustrated in FIG. 7C, in a case where the selection is based on the distribution degree of luminance values, the taken image closer to the focal point can be selected than in a case where the selection is based on the variance value.

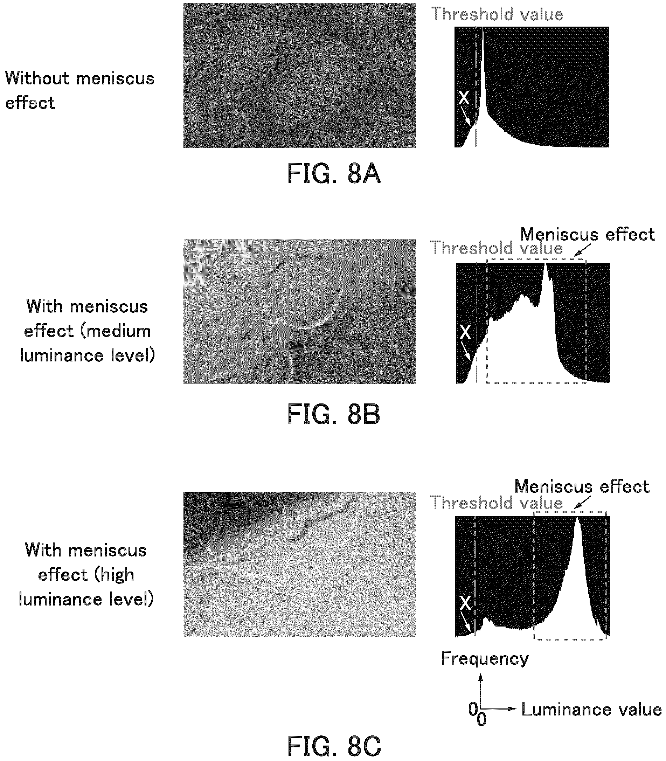

FIG. 8A to FIG. 8C illustrate in-focus images taken in a region unaffected by the meniscus and regions where the meniscus effect differs, and histograms of luminance values of the in-focus images. **In** FIG. 8A shows the result of the in-focus image taken in the region unaffected by the meniscus, FIG. 8B shows the result of the in-focus image taken in the region where the meniscus effect occurs at a medium luminance level, and FIG. 8C shows the result of the in-focus image taken in the region where the meniscus effect occurs at a high luminance level. **In** FIG. 8A to FIG. 8C, the left column shows the in-focus image and the right column illustrates the histogram of each in-focus image. As illustrated in FIG. 8A to FIG. 8C, irrespective of the meniscus effect, the spread of the tail on the low-luminance side of the luminance peak due to a cell was observed, as indicated by the arrow X. Thus, as illustrated in FIG. 8A to FIG. 8C, by setting the threshold value v on the lower luminance side with respect to the peak value of the luminance peak due to a cell, the image closer to the focal position can be selected even when the meniscus effect differs, through the selection based on the distribution degree of luminance values.

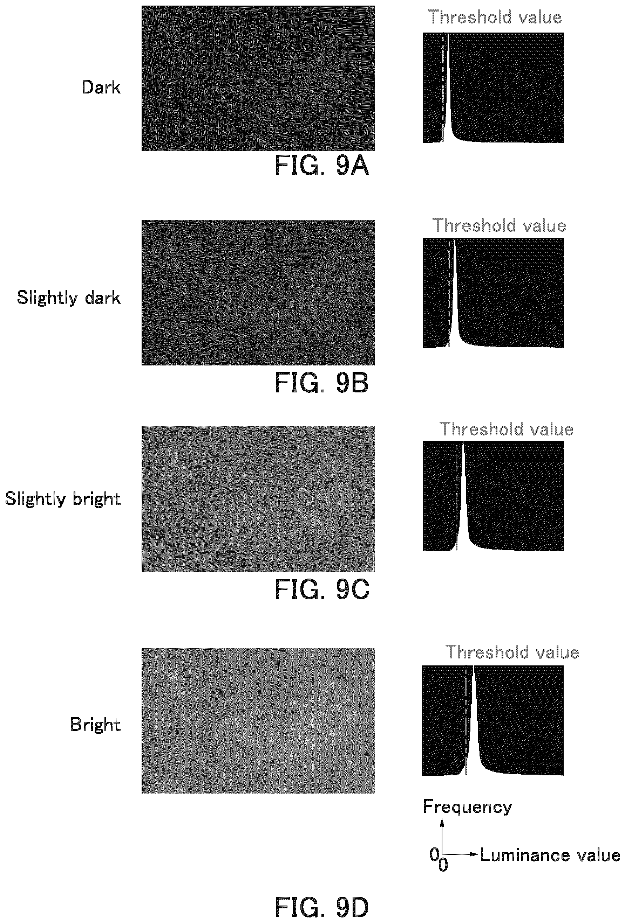

FIG. 9A to 9D illustrate in-focus images taken under conditions with different intensities of illumination light from the illumination 6, and histograms of luminance values of the in-focus images. **In** FIG. 9A to FIG. 9D, the intensity of the illumination light from the illumination 6 becomes stronger in this order. **In** FIG. 9A to 9D, the left column shows the in-focus image, and the right column shows the histogram of each in-focus image. As illustrated in FIG. 9A to 9D, irrespective of the intensity of the illumination light from the illumination 6, the spread of the tail on the low-luminance side of the luminance peak due to a cell was observed. Thus, as illustrated in FIG. 9A to 9D, by setting the threshold value v on the lower luminance side with respect to the peak value of the luminance peak due to a cell, it is possible to select the image closer to the focal position even when the intensity of the illumination light from the illumination 6 differs, through the selection based on the distribution degree of luminance values.

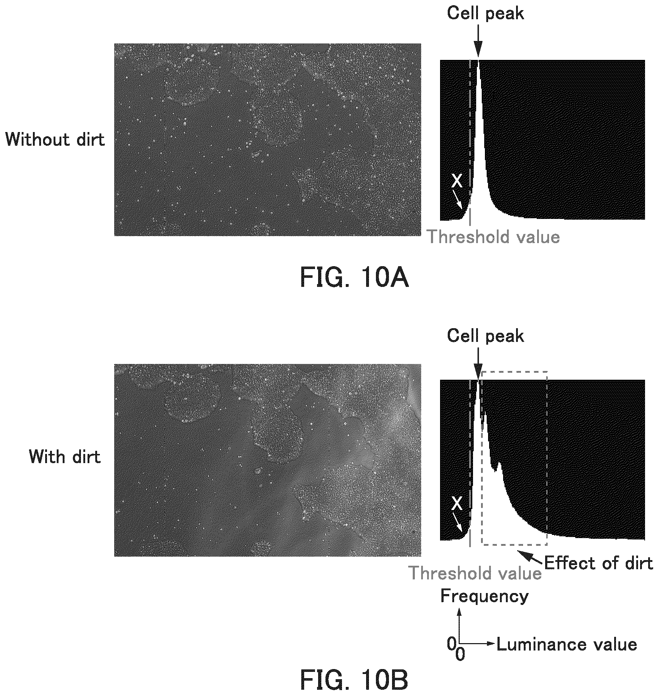

**In** the selection of the in-focus image, a luminance peak due to causes other than the meniscus also affect the selection of the in-focus image. According to the observation apparatus 100 of the present embodiment, optical effects occur in the taken image due to causes other than the meniscus can also be prevented in the selection of the in-focus image. As an example, it will be described that even when there is an effect of dirt adhering to the cell culture vessel, the image closer to the focal position can be selected by evaluating the spread of the tail on the low-luminance side of the luminance peak due to a cell. FIG. 10A to FIG. 10C illustrates in-focus images when there is dirt on the cell culture vessel D and when there is no dirt thereon, and histograms of luminance values of the in-focus images. FIG. 10A shows the result of the in-focus image when there is no dirt, and FIG. 10B shows the result of the in-focus image when there is dirt in the same region as of FIG. 10A. In FIG. 10A to FIG. 10B, the left column shows the in-focus image and the right column illustrates the histogram of each in-focus image. As illustrated in FIG. 10A to FIG. 10C, irrespective of the presence or absence of dirt, the spread of the tail on the low-luminance side of the luminance peak due to a cell was observed, as indicated by the arrow X. Thus, as illustrated in FIG. 10A to FIG. 10B, by setting the threshold value v on the lower luminance side with respect to the peak value of the luminance peak due to a cell, the image closer to the focal position can be selected, irrespective of the presence or absence of dirt, through the selection based on the distribution degree of luminance values.

### (Second Embodiment)

In the first embodiment, the number of pixels with luminance values not more than the threshold value v is evaluated as the distribution degree of luminance values. As illustrated in FIG. 5A and FIG. 5B, and FIG. 6A and FIG. 6B, in taken image closer to the focal position, the tail on the low-luminance side of the luminance peak due to a cell is relatively larger, and thus the minimum luminance value I is relatively lower, that is, the distribution degree of luminance values is relatively higher. Then, in a second embodiment, an observation apparatus that evaluates the minimum luminance values I of the plurality of taken images as the distribution degree of luminance values and selects the taken image closer to the focal position will be described.

According to an observation apparatus 200 of the second embodiment, in step S3, the evaluation unit 513 evaluates the minimum luminance value I of each taken image, and in step S4, the selection unit 514 selects, as the in-focus image, a taken image with the lowest minimum luminance value I. Except for this point, the observation apparatus 200 of the second embodiment has the same configuration as the observation apparatus 100 of the first embodiment, and the description on the configuration of the observation apparatus 100 can also apply to the observation apparatus 200.

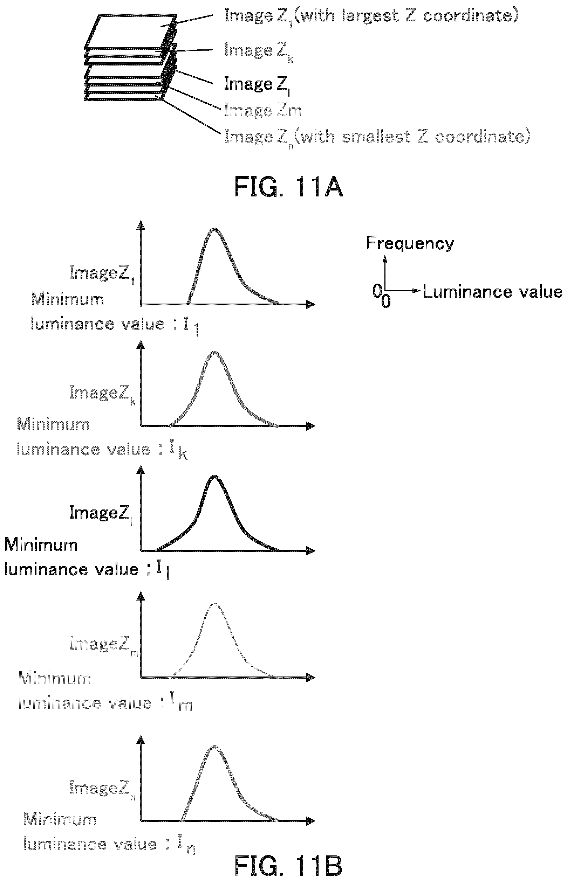

Step S3 and step S4 will be described more specifically using FIG. 11A to FIG.11B. FIG. 11A to FIG.11B is a schematic diagram illustrating an example of histograms of luminance values of a plurality of taken images (Z₁, Zₖ, Zₗ, Zₘ, Zₙ, k < l < m < n, where k, l, m, n are integers of 2 or more) taken at different positions in the Z axis direction. In FIG. 11A to FIG.11B, FIG.11A illustrates the positional relationship in the Z-axis direction of the respective taken images (Z₁ to Zₙ), and FIG.11B illustrates histograms of the respective taken images (Z₁ to Zₙ). As illustrated in FIG. 11A, the respective taken images (Z₁ to Zₙ) are a part of the plurality of taken images, where the image Z₁ refers to the taken image with the largest Z coordinate, the image Zₖ refers to the taken image between the image Z₁ and the image Zₗ, the image Zₗ refers to the in-focus image, and the image Zₘ refers to the taken image between the images Zₗ and Zₙ, and the image Zₙ refers to the taken image with the smallest Z coordinate. As described above, in the taken image closer to the focal position, the tail on the low-luminance side of the luminance peak due to a cell is relatively larger, and thus the minimum luminance value I is relatively lower. Then, in step S3, the distribution degree of luminance values is evaluated by, as illustrated in FIG.11B, extracting the minimum luminance values I (I₁, Iₖ, Iₗ, Iₘ, Iₙ) of the respective taken images (Z₁ to Zₙ).

Specifically, in step S3, the evaluation unit 513 generates, for each taken image (Z₁ to Zₙ), a histogram indicating the distribution of luminance values for all pixels included in the taken image. Then, in step S3, from the histogram of each taken image, the lowest luminance value among luminance values having frequency more than the noise threshold value is extracted as the minimum luminance value I (I₁ to Iₙ), which is minimum luminance information.

Next, in step S4, the selection unit 514 selects the taken image closer to the focal position (in-focus image) using the extracted plurality of minimum luminance values I (I₁ to Iₙ). Specifically, as illustrated in FIG. 11B, the minimum luminance value I is relatively lower in the taken image closer to the focal position. Thus, in step S4, the minimum luminance values I (I₁ to Iₙ) evaluated in step S3 are compared to select the taken image with the lowest minimum luminance value Iₗ as the in-focus image.

In the observation apparatus 200 of the second embodiment, the evaluation unit 513 evaluates the minimum luminance values I in the plurality of taken images, which are unaffected by the meniscus and correlates with the focal position, as the distribution degree of luminance values. Then, in the observation apparatus 200 of the second embodiment, the selection unit 514 selects the taken image closer to the focal position (in-focus image) using the minimum luminance values I. Thus, the observation apparatus 200 of the second embodiment, which is unaffected by the meniscus, can select the taken image closer to the focal position in both of the region affected by the meniscus and the region unaffected by the meniscus. In addition, the observation apparatus 200 of the second embodiment can select the taken image closer to the focal position without setting of the threshold value v.

### (Third Embodiment)

In the first embodiment, the minimum luminance values I of the taken image with the largest Z coordinate and the taken image with the smallest Z coordinate among the plurality of taken images are compared to set, as the threshold value v, the higher of the minimum luminance values. As described above, according to the present invention, the taken image closer to the focal position (in-focus image) can be selected by evaluating the spread of the tail on the low-luminance side of the luminance peak due to a cell. Thus, the threshold value v can also be set based on the peak value (cell peak) in the luminance peak due to a cell. Then, in the third embodiment, an observation apparatus that can select the taken image closer to the focal position (in-focus image) based on the threshold value v set using the cell peak after detecting the luminance peak due to a cell will be described.

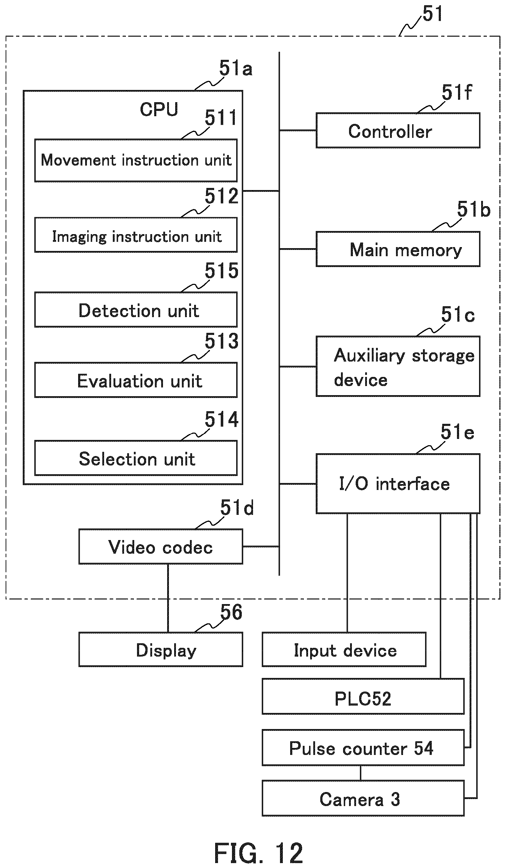

FIG. 12 illustrates an example of a configuration of an observation apparatus 300 of the third embodiment. As illustrated in FIG. 12, the observation apparatus 300 of the third embodiment further includes a detection unit 515 in the arithmetic unit 51 of the observation apparatus 100 of the first embodiment. Except for this point, the observation apparatus 300 of the third embodiment has the same configuration as the observation apparatus 100 of the first embodiment, and the description on the configuration of the observation apparatus 100 can also apply to the observation apparatus 300.

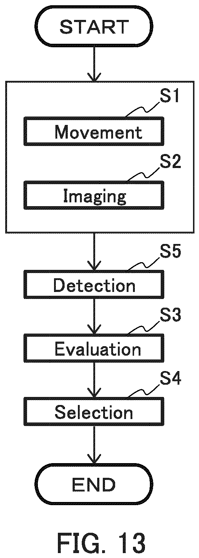

FIG. 13 is a flowchart of an imaging method using the observation apparatus 300 of the third embodiment. As illustrated in FIG. 13, the imaging method of the third embodiment includes step S5 (detection) in addition to the imaging method of the first embodiment, and step S5 is performed after step S2.

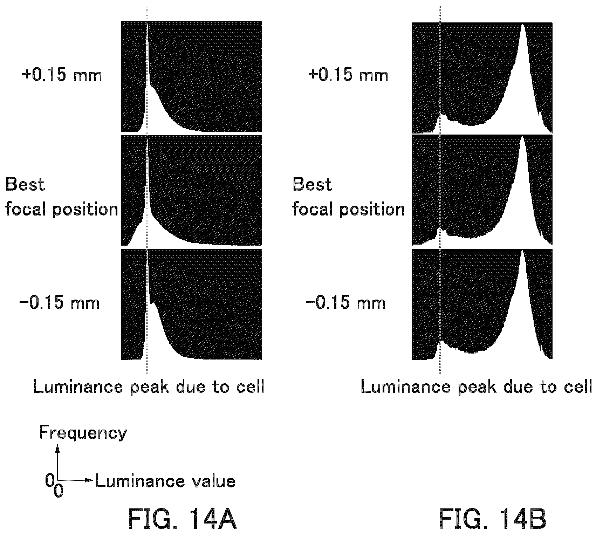

The detection unit 515 detects the cell peak using one or more taken images among the plurality of taken images. The processing by the detection unit 515 in step S5 will be described more specifically using FIG. 14A and FIG. 14B. As illustrated in FIG. 14A and FIG. 14B, the cell peaks (luminance values corresponding to local maximum) in the plurality of taken images are substantially the same, irrespective of the meniscus effect. In addition, as illustrated in FIG. 14A and FIG. 14B, the cell peak is the luminance value corresponding to the lowest local maximum among the plurality of luminance value peaks in each taken image. In step S5, the detection unit 515 first generates a histogram for one or more taken images among the plurality of taken images. Then, in step S5, the local maximum value is detected from the histogram, and then the local maximum value with the lowest luminance value is detected among one or more detected local maximum values, as the cell peak. In the detection of the local maximum value peak, it is preferable to remove noise not more than the noise threshold value in advance.

Next, in step S3, using the luminance value of the cell peak as the threshold value v, the distribution degrees of luminance values is evaluated for the plurality of taken images, that is, the numbers of pixels of luminance values not more than the threshold value v in the plurality of taken images are counted. Although the cell peak is used as the threshold value v in the present embodiment, the threshold value v can be any luminance value between the cell peak and the minimum luminance value I. As a specific example, the threshold value v may be set to a value such as, for example, a value obtained by subtracting, from the cell peak, a prespecified value (for example, 10, 20, or the like), or a value obtained by subtracting, from the cell peak luminance value, a numerical value obtained by multiplying the difference in luminance values between the cell peak and the minimum luminance value I by a predetermined percentage (for example, 1/2, 1/3, 1/4, or the like).

Then, in step S4, the taken image with the largest number of pixels with luminance values not more than the threshold value v is selected as the taken image with the highest distribution degree of luminance values.

In the observation apparatus 300 of the third embodiment, the cell peak can be detected in the processing by the arithmetic unit 51 of the observation apparatus 300, so that the taken image with the highest distribution degree of luminance values can be selected by calculating the threshold value v based on the cell peak. Thus, in the observation apparatus 300 of the third embodiment, there is no need to set the threshold value v, and the in-focus image can be suitably selected even when taking an image of a cell or the like to be measured for the first time.

### (Fourth Embodiment)

In the first embodiment, the threshold value v is set for each divided region, and different threshold values v are used for the respective divided region for the evaluation and the selection. As illustrated in FIG. 14A and FIG. 14B, when images of the identical cell culture vessel D are taken under the same conditions, the luminance peak caused by a cell and the cell peak are substantially the same. Then, in a fourth embodiment, an observation apparatus that sets the threshold value v common for the respective divided region for the evaluation will be described.

In an observation apparatus 400 of the fourth embodiment, in step S3, the evaluation unit 513 sets the threshold value v using the plurality of taken images acquired in one divided region among the plurality of divided regions. Except for this point, the observation apparatus 400 of the fourth embodiment has the same configuration as the observation apparatus 100 of the first embodiment, and the description on the configuration of the observation apparatus 100 can also apply to the observation apparatus 400.

In step S3, the divided region used for setting the threshold value v may be a divided region within the regions unaffected by the meniscus, a divided region within the regions affected by the meniscus, or a divided region spanning both regions. However, for easy setting of the threshold value v, it is preferable to use the divided region within the regions unaffected by the meniscus. Then in the observation apparatus 400 of the fourth embodiment, the threshold value v is set in the same manner as in the observation apparatus 100 of the first embodiment, by comparing the minimum luminance value I₁ in the taken image with the largest Z coordinate and the minimum luminance value I₄ in the taken image with the smallest Z coordinate. Next, in step S3, the number of pixels with luminance values not more than the threshold value v in the taken image is counted using the plurality of taken images for each divided region and the threshold value v.

In the observation apparatus 400 of the fourth embodiment, the threshold value v obtained in one divided region is used, so that the in-focus image can be selected without setting the threshold value v for each division region. Thus, according to the observation apparatus 400 of the fourth embodiment, the processing for acquiring the in-focus image of the entire cell culture vessel D can be reduced, and the in-focus image of the entire cell culture vessel D can be acquired in a shorter time, accordingly.

Although in the observation apparatus 400 of the fourth embodiment, the threshold value v is set through the same processing as in the observation apparatus 100 of the first embodiment, the threshold value v may be set through the same processing as in the observation apparatus 300 of the third embodiment.

### (Fifth Embodiment)

In the first embodiment, the threshold value v is set from the plurality of taken images, and the threshold value v is used for the evaluation and the selection. On the other hand, under the same imaging conditions for the cell culture vessel D, the luminance peaks caused by a cell occur in approximately the same numerical range of luminance values. Thus, the threshold value v for the obtained luminance peak caused by a cell can be set in advance for each of the imaging conditions, to select the in-focus image using the threshold value v. Then, in a fifth embodiment, an observation apparatus that prepares in advance a table of the threshold value v associated with the imaging conditions of the cell culture vessel D, reads out the threshold value v from the imaging conditions associated with the plurality of taken images in the processing by the arithmetic unit 51 to select the in-focus image based on the threshold v will be described.

In an observation apparatus 500 of the fifth embodiment, in addition to the plurality of the taken images, the imaging conditions of the plurality of taken images are stored in step S2, and the threshold value v is set in step S3, from the imaging conditions and the table in which the imaging conditions and the threshold value v are associated with each other. Except for this point, the observation apparatus 500 of the fifth embodiment has the same configuration as the observation apparatus 100 of the first embodiment, and the description on the configuration of the observation apparatus 100 can also apply to the observation apparatus 500.

In the observation apparatus 500 of the fifth embodiment, the table is created prior to the processing by the observation apparatus 500. The table can be created as follows. First, the plurality of taken images are acquired under different imaging conditions using the observation apparatus 500. Next, the threshold value v is set from the acquired plurality of taken images in the same manner as in the observation apparatus 100 of the first embodiment. Then, the imaging conditions and the threshold value are associated with each other and stored in the auxiliary storage device 51c. The imaging conditions include, for example, the intensity of the illumination light of the illumination 6, and the gain value and the exposure time of the camera 3.

Next, in step S3, the evaluation unit 513 acquires and sets the threshold value v associated with the imaging conditions from the imaging conditions and the table that are stored in the auxiliary memory device 51c. Next, in step S3, the number of pixels with luminance values not more than the threshold value v in the taken image is counted using the plurality of taken images of each divided region and the threshold value v.

Since the observation apparatus 500 of the fifth embodiment uses the threshold value v associated with the imaging conditions, the in-focus image can be selected without setting the threshold value v for each of the plurality of taken images. Thus, according to the observation apparatus 500 of the fifth embodiment, the in-focus image can be acquired at a higher speed.

Although the present invention has been described above with reference to the above-described embodiments, the present invention is not limited thereto. Various changes and modifications that may become apparent to those skilled in the art may be made in the configuration and specifics of the present invention within the scope of the present invention.

This application is based upon and claims the benefit of priority from Japanese Patent Application No. 2022-113571 filed on July 15, 2022, the entire disclosure of which is incorporated herein in its entirety by reference.

### <Supplementary Notes>

The whole or part of the exemplary embodiments and examples disclosed above can be described as, but not limited to, the following supplementary notes.

### < Cell Observation Apparatus>

### (Supplementary Note 1)

A cell observation apparatus including:
a placement unit on which a cell culture vessel containing a cell, as an object to be observed, is placed;
an imaging optical system that forms an observation image of the object to be observed;
a first movement unit that moves at least one of the placement unit and the imaging optical system along an optical axis direction of the imaging optical system from a movement start position to a movement end position;
an imaging unit that takes a plurality of images of the observation image in movement in the optical axis direction using an image sensor;
an evaluation unit that evaluates a plurality of taken images based only on a distribution degree of luminance values within a predetermined luminance value range; and
a selection unit that selects a taken image with the highest distribution degree of luminance values as an in-focus image.

### (Supplementary Note 2)

The cell observation apparatus according to Supplementary Note 1, wherein
the evaluation unit measures the number of pixels with a luminance value not more than a threshold value v that is predetermined for the plurality of taken images, and
the selection unit selects a taken image with the largest number of pixels that has been measured.

### (Supplementary Note 3)

The cell observation apparatus according to Supplementary Note 2, wherein
the evaluation unit generates the threshold value v using at least one of the plurality of taken images.

### (Supplementary Note 4)

The cell observation apparatus according to Supplementary Note 3, wherein
the evaluation unit generates, for at least one of the plurality of taken images, a histogram indicating distribution of luminance values for all pixels included in the taken image, and determines the threshold value v using the histogram that has been generated.

### (Supplementary Note 5)

The cell observation apparatus according to Supplementary Note 4, wherein
the evaluation unit extracts a minimum luminance value for each of the plurality of taken images, and sets, to the threshold value v, a highest minimum luminance value among a plurality of extracted minimum luminance values.

### (Supplementary Note 6)

The cell observation apparatus according to Supplementary Note 4, wherein
the evaluation unit compares a lowest luminance value in a taken image at the movement start position and a lowest luminance value in a taken image at the movement end position to set a higher luminance value to the threshold value v.

### (Supplementary Note 7)

The cell observation apparatus according to Supplementary Note 5 or Supplementary Note 6, wherein
the evaluation unit removes, as noise, a luminance value not more than a predetermined number of pixels when measuring the number of pixels with a luminance value not more than the threshold value v, and
the selection unit selects the in-focus image based only on a distribution degree of luminance values not more than a predetermined luminance value from which the noise has been removed.

### (Supplementary Note 8)

The cell observation apparatus according to Supplementary Note 2, further including:
an illumination unit that is arranged to face to the imaging optical system and the image sensor with the placement unit in between, whereinthe illumination unit is configured to be capable of illuminating the cell culture vessel containing the cell and culture solution.

### (Supplementary Note 9)

The cell observation apparatus according to Supplementary Note 8, wherein
the imaging optical system forms, for each of divided regions into which the object to be observed is divided, an observation image of the object to be observed corresponding to the divided region as a divided image, and
the selection unit selects a taken image with the largest number of pixels for the divided image for each of the divided regions.

### (Supplementary Note 10)

The cell observation apparatus according to Supplementary Note 9, wherein
the imaging optical system forms, for a divided region affected by a meniscus that occurs in the cell culture vessel and a divided region unaffected by the meniscus, the observation image of the object to be observed corresponding to the divided region as the divided image.

### (Supplementary Note 11)

The cell observation apparatus according to Supplementary Note 9, further including:
a second movement unit that is capable of moving at least one of the placement unit and the imaging optical system on a plane direction orthogonal to the optical axis direction, wherein
the imaging unit takes a plurality of images at any position on the plane direction, and
the selection unit selects the in-focus image.

### (Supplementary Note 12)

The cell observation apparatus according to Supplementary Note 11, further including:
an integration unit that integrates the in-focus images of the divided regions to generate an in-focus image of an entirety of the cell culture vessel, wherein
the imaging unit takes a plurality of images for all of the divided regions using the second movement unit,
the selection unit selects the in-focus image for all of the divided regions of the cell culture vessel, and
the integration unit integrates the in-focus images that have been selected to generate the in-focus image of the entirety of the cell culture vessel.

### (Supplementary Note 13)

The cell observation apparatus according to Supplementary Note 11, wherein
the first movement unit changes a movement direction along the optical axis direction for each divided region, and
the first movement unit changes the movement direction along the optical axis direction from the movement direction in a previous divided region upon movement to a new divided region by the second movement unit.

### (Supplementary Note 14)

The cell observation apparatus according to Supplementary Note 1 or Supplementary Note 8, wherein
the cell observation device is a phase-contrast microscope.

### (Supplementary Note 15)

A cell observation apparatus including:
a placement unit on which a cell culture vessel containing a cell, as an object to be observed, is placed;
an imaging optical system that forms an observation image of the object to be observed;
a first movement unit that moves at least one of the placement unit and the imaging optical system along an optical axis direction of the imaging optical system from a movement start position to a movement end position;
an imaging unit that takes a plurality of images of the observation image in movement along the optical axis direction using an image sensor;
an evaluation unit that evaluates distribution of luminance values for a plurality of taken images and extracts minimum luminance information related to a minimum luminance value; and
a selection unit that selects an in-focus image based on a plurality of pieces of the minimum luminance information.

### (Supplementary Note 16)

The cell observation apparatus according to Supplementary Note 15, wherein
the evaluation unit generates, for each of the plurality of taken images, a histogram indicating distribution of luminance values for all pixels included in the taken image to evaluate the histogram that has been generated and extract the minimum luminance information.

### (Supplementary Note 17)

The cell observation apparatus according to Supplementary Note 16, wherein
the evaluation unit removes, as noise, a luminance value not more than a predetermined number of pixels when evaluating the histogram that has been generated, and extracts a minimum luminance value in a histogram in which the noise has been removed, as the minimum luminance information.

### (Supplementary Note 18)

The cell observation apparatus according to Supplementary Note 17, further including:
an illumination unit that is arranged to face to the imaging optical system and the image sensor with the placement unit in between, wherein
the illumination unit is configured to be capable of illuminating the cell culture vessel containing the cell and culture solution.

### (Supplementary Note 19)

The cell observation apparatus according to Supplementary Note 18, wherein
the imaging optical system forms, for each of divided regions into which the object to be observed is divided, an observation image of the object to be observed corresponding to the divided region as a divided image, and
the selection unit selects a taken image with the largest number of pixels for the divided image for each of the divided regions.

### (Supplementary Note 20)

the imaging optical system forms, for a divided region affected by a meniscus that occurs in the cell culture vessel and a divided region unaffected by the meniscus, the observation image of the object to be observed corresponding to the divided region as the divided image.

### (Supplementary Note 21)

The cell observation apparatus according to Supplementary Note 20, further including:
a second movement unit that is capable of moving at least one of the placement unit and the imaging optical system on a plane direction orthogonal to the optical axis direction, in which
the imaging unit takes a plurality of images at any position on the plane direction, and the selection unit selects the in-focus image.

### (Supplementary Note 22)

The cell observation apparatus according to Supplementary Note 21, further including:
an integration unit that integrates the in-focus images of the divided regions to generate an in-focus image of an entirety of the cell culture vessel, wherein
the imaging unit takes a plurality of images for all of the divided regions using the second movement unit,
the selection unit selects the in-focus image for all of the divided regions of the cell culture vessel, and
the integration unit integrates the in-focus images that have been selected to generate the in-focus image of the entirety of the cell culture vessel.

### (Supplementary Note 23)

The cell observation apparatus according to Supplementary Note 22, wherein
the first movement unit changes a movement direction along the optical axis direction for each divided region, and
the first movement unit changes the movement direction along the optical axis direction from the movement direction in a previous divided region upon movement to a new divided region by the second movement unit.

### (Supplementary Note 24)

The cell observation apparatus according to Supplementary Note 15, wherein
the cell observation device is a phase-contrast microscope.

### <Imaging Method Used in Cell Observation Apparatus>

### (Supplementary Note 25)

An imaging method used in a cell observation apparatus that includes a placement unit, an imaging optical system, a first movement unit, and an imaging unit, the imaging method including:
an image forming step of, for a cell culture vessel containing a cell that is placed on the placement unit as an object to be observed, forming an image of the object to be observed using the imaging optical system that forms an observation image of the object to be observed;
a moving step of moving at least one of the placement unit and the imaging optical system in an optical axis direction of the imaging optical system from a movement start position to a movement end position;
an imaging step of taking a plurality of images of the observation image in movement along the optical axis direction using an image sensor of the imaging unit;
an evaluating step of evaluating a plurality of taken images based only on a distribution degree of luminance values within a predetermined luminance value range; and
a selecting step of selecting a taken image with the highest distribution degree of luminance values as an in-focus image.

### (Supplementary Note 26)

The imaging method according to Supplementary Note 25, wherein
in the evaluating step, the number of pixels with a luminance value not more than a threshold value v that is predetermined is measured for the plurality of taken images, and
in the selecting step, a taken image with the largest number of pixels that has been measured is selected.

### (Supplementary Note 27)

The imaging method according to Supplementary Note 26, wherein
in the evaluating step, the threshold value v is generated using at least one of the plurality of taken images.

### (Supplementary Note 28)

The imaging method according to Supplementary Note 27, wherein
in the evaluating step, for at least one of the plurality of taken images, a histogram indicating distribution of luminance values for all pixels included in the taken image is generated, and the threshold value v is determined using the histogram that has been generated.

### (Supplementary Note 29)

The imaging method according to Supplementary Note 28, wherein
in the evaluating step, a minimum luminance value is extracted for each of the plurality of taken images, and a highest minimum luminance value among a plurality of extracted minimum luminance values is set to the threshold value v.

### (Supplementary Note 30)

The imaging method according to Supplementary Note 28, wherein
in the evaluating step, a lowest luminance value in a taken image at the movement start position and a lowest luminance value in a taken image at the movement end position are compared, and a higher luminance value is set to the threshold value v.

### (Supplementary Note 31)

The imaging method according to Supplementary Note 29 or Supplementary Note 30, wherein
in the evaluating step, a luminance value not more than a predetermined number of pixels is removed as noise when measuring the number of pixels with a luminance value not more than the threshold value v, and
in the selecting step, the in-focus image is selected based only on a distribution degree of luminance values not more than a predetermined luminance value from which the noise has been removed.

### (Supplementary Note 32)

The imaging method according to Supplementary Note 26, wherein
the cell observation apparatus further includes an illumination unit that is arranged to face to the imaging optical system and the image sensor with the placement unit in between, and
the illumination unit illuminates the cell culture vessel containing the cell and culture solution.

### (Supplementary Note 33)

The imaging method according to Supplementary Note 32, wherein
the imaging optical system forms, for each of divided regions into which the object to be observed is divided, an observation image of the object to be observed corresponding to the divided region as a divided image, and
in the selecting step, a taken image with the largest number of pixels is selected for the divided image for each of the divided regions.

### (Supplementary Note 34)

The imaging method according to Supplementary Note 33, wherein
the imaging optical system forms, for a divided region affected by a meniscus that occurs in the cell culture vessel and a divided region unaffected by the meniscus, the observation image of the object to be observed corresponding to the divided region as the divided image.

### (Supplementary Note 35)

The imaging method according to Supplementary Note 33, wherein
the cell observation apparatus further includes a second movement unit that is capable of moving at least one of the placement unit and the imaging optical system on a plane direction orthogonal to the optical axis direction,
in the imaging step, a plurality of images is taken at any position on the plane direction, and
in the selection step, the in-focus image is selected.

### (Supplementary Note 36)

The imaging method according to Supplementary Note 35, further including:
an integrating step of integrating the in-focus images of the divided regions to generate an in-focus image of an entirety of the cell culture vessel, wherein
in the imaging step, the imaging unit takes a plurality of images for all of the divided regions using the second movement unit,
in the selecting step, the in-focus image is selected for all of the divided regions of the cell culture vessel, and
in the integrating step, the in-focus images that have been selected are integrated to generate the in-focus image of the entirety of the cell culture vessel.

### (Supplementary Note 37)

The imaging method according to Supplementary Note 35, wherein
the first movement unit changes a movement direction along the optical axis direction for each divided region, and
the first movement unit changes the movement direction along the optical axis direction from the movement direction in a previous divided region upon movement to a new divided region by the second movement unit.

### (Supplementary Note 38)

The imaging method according to Supplementary Note 25 or 32, wherein
the cell observation device is a phase-contrast microscope.

### (Supplementary Note 39)

An imaging method used in a cell observation apparatus that includes a placement unit, an imaging optical system, a first movement unit, and an imaging unit, the imaging method including:
an image forming step of, for a cell culture vessel containing a cell that is placed on the placement unit as an object to be observed, forming an image of the object to be observed using the imaging optical system that forms an observation image of the object to be observed;
a moving step of moving at least one of the placement unit and the imaging optical system along an optical axis direction of the imaging optical system from a movement start position to a movement end position;
an imaging step of taking a plurality of images of the observation image in movement in the optical axis direction using an image sensor of the imaging unit;
an evaluating step of evaluating distribution of luminance values for a plurality of taken images and extracting minimum luminance information related to a minimum luminance value; and
a selecting step of selecting an in-focus image based on a plurality of pieces of the minimum luminance information.

### (Supplementary Note 40)

The imaging method according to Supplementary Note 39, wherein
in the evaluating step, for each of the plurality of taken images, a histogram indicating distribution of luminance values for all pixels included in the taken image is generated, the histogram is evaluated, and the minimum luminance information is extracted.

### (Supplementary Note 41)

The imaging method according to Supplementary Note 40, wherein
in the evaluating step, a luminance value not more than a predetermined number of pixels is removed as noise when evaluating the histogram that has been generated, and a minimum luminance value in a histogram in which the noise has been removed is extracted as the minimum luminance information.

### (Supplementary Note 42)

The imaging method according to Supplementary Note 41, wherein
the cell observation apparatus further includes an illumination unit that is arranged to face to the imaging optical system and the image sensor with the placement unit in between, and
the illumination unit is configured to be capable of illuminating the cell culture vessel containing the cell and culture solution.

### (Supplementary Note 43)

The imaging method according to Supplementary Note 42, wherein
the imaging optical system forms, for each of divided regions into which the object to be observed is divided, an observation image of the object to be observed corresponding to the divided region as a divided image, and
in the selecting step, a taken image with the largest number of pixels is selected for the divided image for each of the divided regions.

### (Supplementary Note 44)

The imaging method according to Supplementary Note 43, wherein
the imaging optical system forms, for a divided region affected by a meniscus that occurs in the cell culture vessel and a divided region unaffected by the meniscus, the observation image of the object to be observed corresponding to the divided region as the divided image.

### (Supplementary Note 45)

The imaging method according to Supplementary Note 44, wherein
the cell observation apparatus further includes a second movement unit that is capable of moving at least one of the placement unit and the imaging optical system on a plane direction orthogonal to the optical axis direction,
in the imaging step, a plurality of images is taken at any position on the plane direction, and
in the selection step, the in-focus image is selected.

### (Supplementary Note 46)

The imaging method according to Supplementary Note 45, further including:
an integrating step of integrating the in-focus images of the divided regions to generate an in-focus image of an entirety of the cell culture vessel, wherein
in the imaging step, the imaging unit takes a plurality of images for all of the divided regions using the second movement unit,
in the selecting step, the in-focus image is selected for all of the divided regions of the cell culture vessel, and
in the integrating step, the in-focus images that have been selected are integrated to generate the in-focus image of the entirety of the cell culture vessel.

### (Supplementary Note 47)

The imaging method according to Supplementary Note 46, wherein
the first movement unit changes a movement direction along the optical axis direction for each divided region, and
the first movement unit changes the movement direction along the optical axis direction from the movement direction in a previous divided region upon movement to a new divided region by the second movement unit.

### (Supplementary Note 48)

The imaging method according to Supplementary Note 39, wherein
the cell observation device is a phase-contrast microscope.

### REFERENCE SIGNS LIST

- 1:: stage
- 2:: objective lens
- 3:: camera
- 4:: movement unit
- 41:: Z-axis direction movement unit
- 41a:: Z-axis motor
- 41b:: Z-axis ball screw
- 42:: X-axis direction movement unit
- 42a:: X-axis motor
- 42b:: X-axis ball screw
- 43:: Y-axis motor
- 43a:: Y-axis motor
- 43b:: Y-axis ball screw
- 5:: control unit
- 51:: arithmetic unit
- 51a:: CPU
- 511:: movement instruction unit
- 512:: imaging instruction unit
- 513:: evaluation unit
- 514:: selection unit
- 515:: detection unit
- 51b:: main memory
- 51c:: auxiliary storage device
- 51d:: video codec
- 51e:: I/O interface
- 51f:: controller
- 52:: PLC
- 53:: motor driver
- 53x:: X-axis direction motor driver
- 53y:: Y-axis direction motor driver
- 53z:: Z-axis direction motor driver
- 54:: pulse counter
- 56:: display device
- 6:: illumination
- 61:: light source
- 62:: support member
- 100, 200, 300, 400, 500:: observation apparatus

## Claims

1. A cell observation apparatus comprising:
a placement unit on which a cell culture vessel containing a cell, as an object to be observed, is placed;
an imaging optical system that forms an observation image of the object to be observed;
a first movement unit that moves at least one of the placement unit and the imaging optical system along an optical axis direction of the imaging optical system from a movement start position to a movement end position;
an imaging unit that takes a plurality of images of the observation image in movement in the optical axis direction using an image sensor;
an evaluation unit that evaluates a plurality of taken images based only on a distribution degree of luminance values within a predetermined luminance value range; and
a selection unit that selects a taken image with a highest distribution degree of luminance values as an in-focus image.

2. The cell observation apparatus according to claim 1, wherein
the evaluation unit measures the number of pixels with a luminance value not more than a threshold value v that is predetermined for the plurality of taken images, and
the selection unit selects a taken image with a largest number of pixels that has been measured.

3. The cell observation apparatus according to claim 2, wherein
the evaluation unit generates the threshold value v using at least one of the plurality of taken images.

4. The cell observation apparatus according to claim 3, wherein
the evaluation unit generates, for at least one of the plurality of taken images, a histogram indicating distribution of luminance values for all pixels included in the taken image, and determines the threshold value v using the histogram that has been generated.

5. The cell observation apparatus according to claim 4, wherein
the evaluation unit extracts a minimum luminance value for each of the plurality of taken images, and sets, to the threshold value v, a highest minimum luminance value among a plurality of extracted minimum luminance values.

6. The cell observation apparatus according to claim 4, wherein
the evaluation unit compares a lowest luminance value in a taken image at the movement start position and a lowest luminance value in a taken image at the movement end position to set a higher luminance value to the threshold value v.

7. The cell observation apparatus according to claim 5 or 6, wherein
the evaluation unit removes, as noise, a luminance value not more than a predetermined number of pixels when measuring the number of pixels with a luminance value not more than the threshold value v, and
the selection unit selects the in-focus image based only on a distribution degree of luminance values not more than a predetermined luminance value from which the noise has been removed.

8. The cell observation apparatus according to claim 2, further comprising:
an illumination unit that is arranged to face to the imaging optical system and the image sensor with the placement unit in between, wherein
the illumination unit is configured to be capable of illuminating the cell culture vessel containing the cell and culture solution.

9. The cell observation apparatus according to claim 8, wherein
the imaging optical system forms, for each of divided regions into which the object to be observed is divided, an observation image of the object to be observed corresponding to the divided region as a divided image, and
the selection unit selects a taken image with a largest number of pixels for the divided image for each of the divided regions.

10. The cell observation apparatus according to claim 9, wherein
the imaging optical system forms, for a divided region affected by a meniscus that occurs in the cell culture vessel and a divided region unaffected by the meniscus, the observation image of the object to be observed corresponding to the divided region as the divided image.

11. The cell observation apparatus according to claim 9, further comprising:
a second movement unit that is capable of moving at least one of the placement unit and the imaging optical system on a plane direction orthogonal to the optical axis direction, wherein
the imaging unit takes a plurality of images at any position on the plane direction, and
the selection unit selects the in-focus image.

12. The cell observation apparatus according to claim 11, further comprising:
an integration unit that integrates the in-focus images of the divided regions to generate an in-focus image of an entirety of the cell culture vessel, wherein
the imaging unit takes a plurality of images for all of the divided regions using the second movement unit,
the selection unit selects the in-focus image for all of the divided regions of the cell culture vessel, and
the integration unit integrates the in-focus images that have been selected to generate the in-focus image of the entirety of the cell culture vessel.

13. The cell observation apparatus according to claim 11, wherein
the first movement unit changes a movement direction along the optical axis direction for each divided region, and
the first movement unit changes the movement direction along the optical axis direction from the movement direction in a previous divided region upon movement to a new divided region by the second movement unit.

14. The cell observation apparatus according to claim 1 or 8, wherein
the cell observation device is a phase-contrast microscope.

15. A cell observation apparatus comprising:
a placement unit on which a cell culture vessel containing a cell, as an object to be observed, is placed;
an imaging optical system that forms an observation image of the object to be observed;
a first movement unit that moves at least one of the placement unit and the imaging optical system along an optical axis direction of the imaging optical system from a movement start position to a movement end position;
an imaging unit that takes a plurality of images of the observation image in movement along the optical axis direction using an image sensor;
an evaluation unit that evaluates distribution of luminance values for a plurality of taken images and extracts minimum luminance information related to a minimum luminance value; and
a selection unit that selects an in-focus image based on a plurality of pieces of the minimum luminance information.

16. An imaging method used in a cell observation apparatus including a placement unit, an imaging optical system, a first movement unit, and an imaging unit, the imaging method comprising:
an image forming step of, for a cell culture vessel containing a cell that is placed on the placement unit as an object to be observed, forming an image of the object to be observed using the imaging optical system that forms an observation image of the object to be observed;
a moving step of moving at least one of the placement unit and the imaging optical system along an optical axis direction of the imaging optical system from a movement start position to a movement end position;
an imaging step of taking a plurality of images of the observation image in movement in the optical axis direction using an image sensor of the imaging unit;
an evaluating step of evaluating a plurality of taken images based only on a distribution degree of luminance values within a predetermined luminance value range; and
a selecting step of selecting a taken image with a highest distribution degree of luminance values as an in-focus image.

17. An imaging method used in a cell observation apparatus that includes a placement unit, an imaging optical system, a first movement unit, and an imaging unit, the imaging method comprising:
an image forming step of, for a cell culture vessel containing a cell that is placed on the placement unit as an object to be observed, forming an image of the object to be observed using the imaging optical system that forms an observation image of the object to be observed;
a moving step of moving at least one of the placement unit and the imaging optical system along an optical axis direction of the imaging optical system from a movement start position to a movement end position;
an imaging step of taking a plurality of images of the observation image in movement in the optical axis direction using an image sensor of the imaging unit;
an evaluating step of evaluating distribution of luminance values for a plurality of taken images and extracting minimum luminance information related to a minimum luminance value; and
a selecting step of selecting an in-focus image based on a plurality of pieces of the minimum luminance information.
